(19) Europäisches Patentamt
European Patent Office
Office européen des brevets

(11) Veröffentlichungsnummer: **0 006 456**
**A1**

# (12) EUROPÄISCHE PATENTANMELDUNG

(21) Anmeldenummer: **79101626.4**

(22) Anmeldetag: **28.05.79**

(51) Int. Cl.³: **C 07 C 69/74**, A 01 N 9/24, C 07 C 121/68

(30) Priorität: **09.06.78 DE 2825314**

(43) Veröffentlichungstag der Anmeldung: **09.01.80 Patentblatt 80/1**

(84) Benannte Vertragsstaaten: **BE CH DE FR GB IT NL**

(71) Anmelder: **BAYER Aktiengesellschaft, Zentralbereich Patente,Marken und Lizenzen Bayerwerk, D-5090 Leverkusen 1 (DE)**

(72) Erfinder: **Fuchs, Rainer, Dr., Roeberstrasse 8, D-5600 Wuppertal 1 (DE)**
Erfinder: **Hammann, Ingeborg, Dr., Belfortstrasse 9, D-5000 Köln 1 (DE)**
Erfinder: **Stendel, Wilhelm, Dr., In den Birken 55, D-5600 Wuppertal 1 (DE)**

## (54) Spiropentancarbonsäureester, Verfahren zu deren Herstellung, insektizide und akarizide Mittel, Verwendung der Spiropentancarbonsäureester zur Bekämpfung von Insekten oder Spinnentieren und Verfahren zur Herstellung insektizider oder akarizider Mittel.

(57) Die Erfindung betrifft, Spiropentancarbonsäureester der Formel

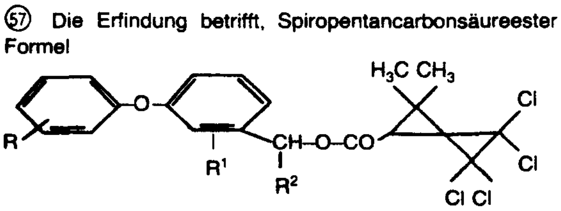
(I)

in welcher R, $R^1$ und $R^2$ die in der Beschreibung angegebene Bedeutung besitzen.

Sie zeichnen sich durch insektizide und akarizide Wirksamkeit aus. Sie werden erhalten, indem man 4,4,5,5-Tetrachloro-2,2-dimethyl-spiropentancarbonsäurechlorid der Formel

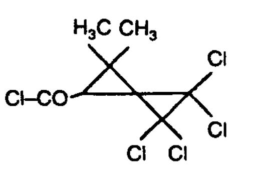
(II)

mit substituierten Phenoxybenzylalkoholen der Formel

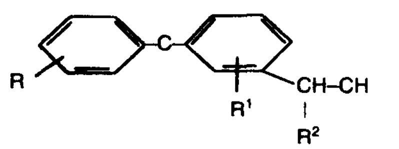
(III)

umsetzt.

EP 0 006 456 A1

0006456



BAYER AKTIENGESELLSCHAFT 5090 Leverkusen, Bayerwerk
Zentralbereich Rt-by
Patente, Marken und Lizenzen Typ Ia

BEZEICHNUNG GEÄNDERT
siehe Titelseite

Substituierte Spiropentancarbonsäureester, Verfahren zu ihrer Herstellung und ihre Verwendung als Insektizide und Akarizide

Die Erfindung betrifft neue Spiropentancarbonsäureester, ein Verfahren zu ihrer Herstellung sowie ihre Verwendung als Insektizide und Akarizide.

Es ist bekannt, daß Phenoxybenzylester von Spirocarbonsäuren, wie z.B. 2,2-Dimethylspiro(2,4) hepta-4,6-dien-1-carbonsäure-m-phenoxybenzylester zur Bekämpfung von Insekten geeignet sind (vergleiche DE-OS 2 605 828). Die Wirkung dieser Verbindungen ist jedoch, insbesondere bei niedrigen Konzentrationen und Aufwandmengen, nicht immer zufriedenstellend.

Es wurden nun die neuen Spiropentancarbonsäureester der Formel (I)

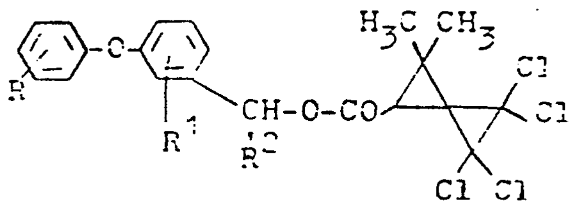

(I)

in welcher

R und $R^1$ gleich oder verschieden sind und für Wasserstoff oder Halogen stehen und

$R^2$ für Wasserstoff, Cyano oder Äthinyl steht,

gefunden.

Die allgemeine Formel (I) schließt die verschiedenen Isomeren und deren Mischungen mit ein.

Die neuen Spiropentancarbonsäureester zeichnen sich durch insektizide und akarizide Wirksamkeit aus.

Weiterhin wurde gefunden, daß man die neuen Spiropentancarbonsäureester der Formel (I) erhält, wenn man 4,4,5,5-Tetrachloro-2,2-dimethyl-spiropentancarbonsäurechlorid der Formel

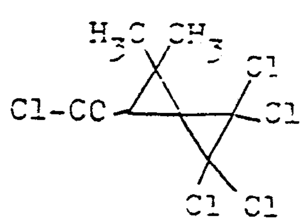

(II)

mit substituierten Phenoxybenzylalkoholen der Formel

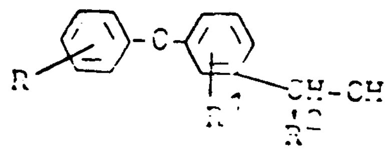

(III)

in welcher

R,$R^1$ und $R^2$ die oben angegebene Bedeutung haben,

gegebenenfalls in Gegenwart eines Säureakzeptors und gegebenenfalls in Gegenwart eines Verdünnungsmittels umsetzt.

Überraschenderweise zeigen die erfindungsgemäßen Spiropentancarbonsäureester eine bessere insektizide Wirkung als die entsprechenden bekannten Produkte analoger Konstitution und gleicher Wirkungsrichtung. Die Produkte gemäß vorliegender Erfindung stellen somit eine wertvolle Bereicherung der Technik dar.

Gegenstand der Erfindung sind vorzugsweise Spiropentancarbonsäureester der Formel (I)

in welcher

R und $R^1$ gleich oder verschieden sind und für Wasserstoff oder Fluor stehen und

$R^2$ für Wasserstoff oder Cyano steht.

Als Beispiele hierfür seien im einzelnen genannt: 4,4,5,5-Tetrachloro-2,2-dimethyl-spiropentan-1-carbonsäure-3-(4-fluoro-phenoxy)-benzylester, -3-(3-fluoro-phenoxy)-benzylester, -3-(2-fluoro-phenoxy)-benzylester, -4-fluoro-3-phenoxy-benzylester, -5-fluoro-3-phenoxy-benzylester, -6-fluoro-3-phenoxy-benzylester und -2-fluoro-3-phenoxy-benzylester sowie 4,4,5,5-Tetrachloro-2,2-dimethyl-spiropentan-1-carbonsäure-3-(4-fluoro-phenoxy)- α -cyano-benzylester, -3-(3-fluoro-phenoxy)- α-cyano-benzylester, -3-(2-fluoro-phenoxy)- α -cyano-benzylester, -4-fluoro-3-phenoxy- α -cyano-benzylester, -5-fluoro-3-phenoxy- α -cyano-benzylester, -6-fluoro-3-phenpxy- α -cyano-benzylester und -2-fluoro-3-phenoxy- α -cyano-benzylester.

Verwendet man beispielsweise 4,4,5,5-Tetrachlor-2,2-dimethylspiropentan-carbonsäurechlorid und 4-Fluoro-3-(4-fluoro-phenoxy)- α -cyano-benzylalkohol als Ausgangsstoffe, so kann der Reaktionsverlauf durch folgendes Formelschema skizziert werden:

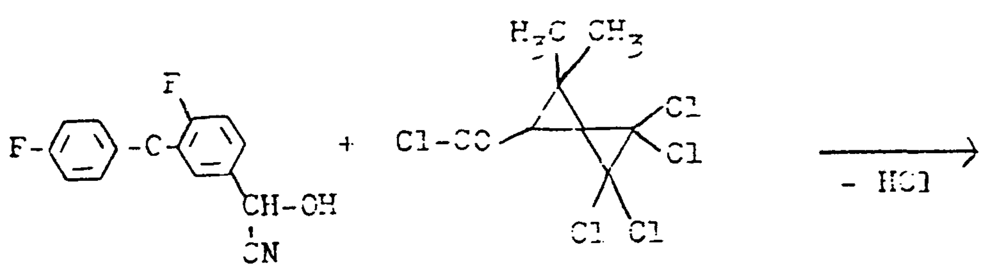

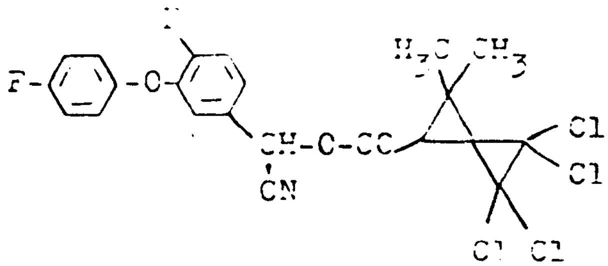

Die zu verwendenden Ausgangsstoffe sind durch die Formeln (II) und (III) definiert.
Vorzugsweise stehen darin

R und $R^1$ für Wasserstoff oder Fluor und

$R^2$ für Wasserstoff oder Cyano.

Das als Ausgangsprodukt zu verwendende 4,4,5,5-Tetrachloro-2,2-dimethyl-spiropentan-carbonsäurechlorid (II) kann ausgehend vom bekannten 4,4,5,5-Tetrachlor-2,2-dimethyl-spiropentan-carbonsäure-methylester (vergleiche Chem.Ber. 107 (1974), 2760; Liebigs Ann. Chem. 758 (1972), 148) nach üblichen Methoden hergestellt werden, indem man beispielsweise zunächst aus dem Ester durch Kochen mit wäßrig-alkoholischer Alkalihydroxidlösung die entsprechende Carbonsäure herstellt. Die Aufarbeitung erfolgt z.B. durch Abdestillieren des Alkohols, Verdünnen mit Wasser, Schütteln mit Methylenchlorid und Ansäuern der wäßrigen Phase mit einer starken Säure, wobei 4,4,5,5-Tetrachloro-2,2-dimethyl-spiropentan-1-carbonsäure als kristallines Produkt anfällt.

Aus der genannten Säure kann das Säurechlorid der Formel (II) auf bekannte Weise durch Umsetzung mit einem Chlorierungsmittel wie z.B. Thionylchlorid, gegebenenfalls unter Verwendung eines Verdünnungsmittels wie

z.B. Tetrachlorkohlenstoff, bei Temperaturen zwischen 20 und 100°C, vorzugsweise zwischen 50 und 90°C, hergestellt werden. Es wird gegebenenfalls durch Vakuumdestillation gereinigt.

Die weiter als Ausgangsverbindungen zu verwendenden Phenoxybenzylalkohole der Formel (III) sind bekannt oder können analog bekannten Verfahren hergestellt werden (vergleiche DE-OS 2 621 433).

Als Beispiele hierfür seien im einzelnen genannt:
3-(4-Fluoro-phenoxy)-, 3-(3-Fluoro-phenoxy)- und 3-(2-Fluoro-phenoxy)-benzylalkohol,
4-Fluoro-3-phenoxy)-, 5-Fluoro-3-phenoxy-, 6-Fluoro-3-phenoxy- und 2-Fluoro-3-phenoxy-benzylalkohol,
3-(4-Fluoro-phenoxy)- α -cyano-benzylalkohol,
3-(3-Fluoro-phenoxy)- α -cyano-benzylalkohol,
3-(2-Fluoro-phenoxy)- α -cyano-benzylalkohol sowie
4-Fluoro-3-phenoxy- α -cyano-benzylalkohol,
5-Fluoro-3-phenoxy- α -cyano-benzylalkohol,
6-Fluoro-3-phenoxy- α -cyano-benzylalkohol und
2-Fluoro-3-phenoxy- α -cyano-benzylalkohol.

Das Verfahren zur Herstellung der erfindungsgemäßen Spiropentancarbonsäureester wird bevorzugt unter Verwendung geeigneter Lösungs- oder Verdünnungsmittel durchgeführt. Als solche kommen praktisch alle inerten organischen Solventien in Frage. Hierzu gehören insbesondere aliphatische und aromatische, gegebenenfalls chlorierte Kohlenwasserstoffe, wie Benzin, Benzol,

Toluol, Xylol, Methylenchlorid, Chloroform, Tetrachlorkohlenstoff, Chlorbenzol und o-Dichlorbenzol, Äther wie Diäthyl- und Dibutyläther, Tetrahydrofuran und Dioxan, Ketone wie Aceton, Methyläthyl-, Methylisopropyl- und Methylisobutylketon sowie Nitrile wie Acetonitril und Propionitril.

Als Säureakzeptoren können alle üblichen Säurebindemittel Verwendung finden. Besonders bewährt haben sich Alkalicarbonate und -alkoholate, wie Natrium- und Kaliumcarbonat, Natrium- und Kaliummethylat bzw. -äthylat, ferner aliphatische, aromatische oder heterocyclische Amine, beispielsweise Triäthylamin, Trimethylamin, Dimethylanilin, Dimethylbenzylamin und Pyridin.

Die Reaktionstemperatur kann innerhalb eines größeren Bereichs variiert werden. Im allgemeinen arbeitet man zwischen 0 und 100°C, vorzugsweise bei 15 bis 40°C. Die Umsetzung wird im allgemeinen bei Normaldruck durchgeführt.

Zur Durchführung des erfindungsgemäßen Verfahrens werden die Ausgangsstoffe gewöhnlich in äquimolaren Mengen eingesetzt. Ein Überschuß der einen oder anderen Reaktionskomponente bringt keine wesentlichen Vorteile. Die Umsetzung wird im allgemeinen in einem geeigneten Verdünnungsmittel in Gegenwart eines Säureakzeptors durchgeführt und das Reaktionsgemisch wird mehrere Stunden bei der erforderlichen Temperatur gerührt.

Danach kühlt man ab, gießt in Wasser und schüttelt mit einem organischen Lösungsmittel, z.B. Toluol aus. Die organische Phase wird dann wie üblich durch Waschen, Trocknen und Abdestillieren des Lösungsmittels aufgearbeitet.

Die neuen Verbindungen fallen in Form von Ölen an, die sich zum Teil nicht unzersetzt destillieren lassen, jedoch durch sogenanntes "Andestillieren", d.h. durch längeres Erhitzen unter vermindertem Druck auf mäßig erhöhte Temperaturen von den letzten flüchtigen Anteilen befreit und auf diese Weise gereinigt werden. Zu ihrer Charakterisierung dient der Brechungsindex.

Die Wirkstoffe eignen sich bei guter Pflanzenverträglichkeit und günstiger Warmblütertoxizität zur Bekämpfung von tierischen Schädlingen, insbesondere Insekten und Spinnentieren, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.
Aus der Ordnung der Diplopoda z.B. Blaniulus guttulatus.
Aus der Ordnung der Chilopoda z.B. Geophilus carpophaus, Scutigera spec.
Aus der Ordnung der Symphyla z.B. Scutigerella immaculata.
Aus der Ordnung der Thysanura z.B. Lepisma saccharina.
Aus der Ordnung der Collembola z.B. Onychiurus armatus.

Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.

Aus der Ordnung der Dermaptera z.B. Forficula auricularia.

Aus der Ordnung der Isoptera z.B. Reticulitermes spp..

Aus der Ordnung der Anoplura z.B. Phylloxera vastatrix, Pemphigus spp., Pediculus humanus corporis, Haematopinus spp., Linognathus spp.

Aus der Ordnung der Mallophaga z.B. Trichodectes spp., Damalinea spp.

Aus der Ordnung der Thysanoptera z.B. Hercinothrips fermoralis, Thrips tabaci.

Aus der Ordnung der Heteroptera z.B. Eurygaster spp., Dysdercus intermedius, Piesma quadrata, Cimex lectularius, Rhodnius prolixus, Triatoma spp.

Aus der Ordnung der Homoptera z.B. Aleurodes brassicae, Bemisia tabaci, Trialeurodes vaporariorum, Aphis gossypii, Brevicoryne brassicae, Cryptomyzus ribis, Doralis fabae, Doralis pomi, Eriosoma lanigerum, Hyalopterus arundinis, Macrosiphum avenae, Myzus spp.,Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.

Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis

citrella, Agrotis spp., Euxoa spp., Feltia spp. Earias insulana, Heliothis spp., Laphygma exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Cacoecia podana, Capua reticulana, Choristoneura fumiferana, Clysia ambiguella, Homona magnanima, Tortrix viridana.
Aus der Ordnung der Coleoptera z.B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.
Aus der Ordnung der Hymenoptera z.B. Diprion spp., Hoplocampa spp., Lasius spp., Monomorium pharaonis, Vespa spp.
Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis,

Ceratophyllus spp..
Aus der Ordnung der Arachnida z.B. Scorpio maurus, Latrodectus mactans.
Aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp..

Die Wirkstoffe können in die üblichen Formulierungen übergeführt werden, wie Lösungen, Emulsionen, Spritzpulver, Suspensionen, Pulver, Stäubemittel, Schäume, Pasten, lösliche Pulver, Granulate, Aerosole, Suspensions-Emulsionskonzentrate, Saatgutpuder, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä. sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine, z.B. Erdölfraktionen, Alkohole, wie Butanol oder Glycol sowie deren Äther und Ester, Ketone, wie Aceton, Methyläthylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgas, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyäthylen-Fettsäure-Ester, Polyoxyäthylen-Fettalkohol-Äther, z.B. Alkylarylpolyglykol-äther, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige,

körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azol-Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die Anwendung der erfindungsgemäßen Wirkstoffe erfolgt in Form ihrer handelsüblichen Formulierungen und/oder den aus diesen Formulierungen bereiteten Anwendungsformen.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 100 Gew.-% Wirkstoff, vorzugsweise zwischen 0,01 und 10 Gew.-% liegen.

Die Anwendung geschieht in einer den Anwendungsformen angepaßten üblichen Weise.

Bei der Anwendung gegen Hygiene- und Vorratsschädlinge zeichnen sich die Wirkstoffe durch eine hervorragende Residualwirkung auf Holz und Ton sowie durch eine gute Alkalistabilität auf gekälkten Unterlagen aus.

Die Anwendung der erfindungsgemäßen Wirkstoffe geschieht im Veterinärsektor in bekannter Weise, wie durch orale Anwendung in Form von beispielsweise Tabletten, Kapseln, Tränken, Granulaten, durch dermale Anwendung in Form beispielsweise des Tauchens (Dippen), Sprühens (Sprayen), Aufgießens (pour-on and spot-on) und des Einpuders sowie durch parenterale Anwendung in Form beispielsweise der Injektion.

Beispiel A

Laphygma-Test

Lösungsmittel: 3 Gewichtsteile Dimethylformamid
Emulgator: 1 Gewichtsteil Alkylarylpolyglykoläther

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Raupen des Eulenfalters (Laphygma frugiperda) besetzt, solange die Blätter noch feucht sind.

Nach der gewünschten Zeit wird die Abtötung in % bestimmt. Dabei bedeuten 100 %, daß alle Raupen abgetötet wurden; 0 % bedeutet, daß keine Raupen abgetötet wurden.

Bei diesem Test zeigen z.B. die folgenden Verbindungen der Herstellungsbeispiele überlegene Wirksamkeit gegenüber dem Stand der Technik: 1

<u>Beispiel B</u>

Myzus-Test

Lösungsmittel: 3 Gewichtsteile Dimethylformamid
Emulgator: 1 Gewichtsteil Alkylarylpolyglykoläther

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea), die stark von der Pfirsichblattlaus (Myzus persicae) befallen sind, werden durch Tauchen in die Wirkstoffzubereitung der gewünschten Konzentration behandelt.

Nach der gewünschten Zeit wird die Abtötung in % bestimmt. Dabei bedeuten 100 %, daß alle Blattläuse abgetötet wurden; 0 % bedeutet, daß keine Blattläuse abgetötet wurden.

Bei diesem Test zeigen z.B. die folgenden Verbindungen der Herstellungsbeispiele überlegene Wirksamkeit gegenüber dem Stand der Technik: 1

Beispiel C

Test mit parasitierenden Fliegenlarven

Emulgator: 80 Gewichtsteile Alkylarylpolyglykoläther

Zur Herstellung einer zweckmäßigen Wirkstoffzubereitung vermischt man 20 Gewichtsteile der betreffenden aktiven Substanz mit der angegebenen Menge des Emulgators und verdünnt das so erhaltene Gemisch mit Wasser auf die gewünschte Konzentration.

Etwa 20 Fliegenlarven (Lucilia cuprina) werden in ein mit Wattestopfen entsprechender Größe beschicktes Teströhrchen gebracht, welches ca. 3 ml einer 20 %igen Eigelbpulver-Suspension in Wasser enthält. Auf diese Eigelbpulver-Suspension werden 0,5 ml der Wirkstoffzubereitung gebracht. Nach 24 Stunden wird der Abtötungsgrad in % bestimmt. Dabei bedeuten 100 %, daß alle und 0 %, daß keine Larben abgetötet worden sind.

Bei diesem Test zeigen z.B. die folgenden Verbindungen der Herstellungsbeispiele überlegene Wirksamkeit gegenüber dem Stand der Technik: 1

Beispiel D

Test mit parasitierenden adulten Rinderzecken (Boophilus microplus res.)

Lösungsmittel: Alkylarylpolyglykoläther

Zur Herstellung einer zweckmäßigen Wirkstoffzubereitung vermischt man die betreffende aktive Substanz mit dem angegebenen Lösungsmittel im Verhältnis 1:2 und verdünnt das so erhaltene Konzentrat mit Wasser auf die gewünschte Konzentration.

10 adulte Rinderzecken (B.microplus res.) werden in der zu testenden Wirkstoffzubereitung 1 Minute getaucht. Nach Überführung in Plastikbecher und Aufbewahrung in einem klimatisierten Raum wird der Abtötungsgrad in Prozent bestimmt, wobei 100 % bedeuten, daß alle und 0 %, daß keine Zecken abgetötet worden sind.

Bei diesem Test zeigen z.B. die folgenden Verbindungen der Herstellungsbeispiele überlegene Wirksamkeit gegenüber dem Stand der Technik: 1

**Herstellungsbeispiele**

**Beispiel 1:**

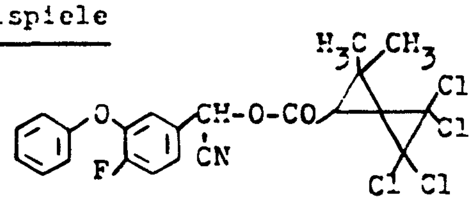

7,5 g (0,025 Mol) 4,4,5,5-Tetrachloro-2,2-dimethylspiropentan-1-carbonsäurechlorid und 6,1 g (0,025 Mol) 3-Phenoxy-4-fluoro-α-cyano-benzylalkohol werden in 100 ml wasserfreiem Toluol gelöst und bei 20-25°C 2,2 g Pyridin, gelöst in 50 ml wasserfreiem Toluol, unter Rühren zugetropft. Anschließend wird weitere 3 Stunden bei 25°C gerührt. Das Reaktionsgemisch wird in 150 ml Wasser gegossen, die organische Phase abgetrennt und nochmals mit 100 ml Wasser gewaschen. Anschließend wird die Toluolphase über Natriumsulfat getrocknet und das Lösungsmittel im Wasserstrahlvakuum abdestilliert. Letzte Lösungsmittelreste werden durch kurzes Andestillieren bei 60°C/1 Torr Badtemperatur entfernt. Man erhält 10,2 g (81,1 % der Theorie) 4,4,5,5-Tetrachloro-2,2-dimethylspiropentan-1-carbonsäure-3-phenoxy-4-fluoro-α-cyano-benzylester als gelbes Öl mit dem Brechungsindex $n_D^{25}$: 1,5653.

Analog Beispiel 1 erhält man:

| | | | |
|---|---|---|---|
| Beispiel 2 | 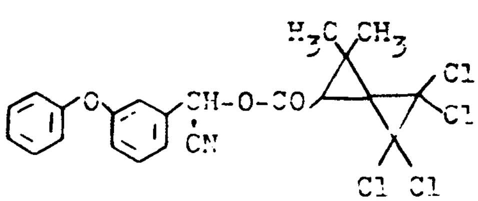 | Ausbeute: 72,7 % der Theorie | Brechungsindex $n_D^{25}$: 1,5641 |

Beispiel 3 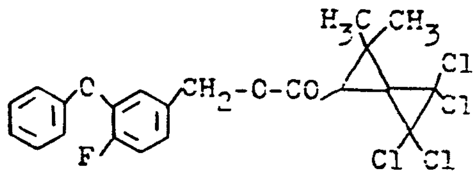 Ausbeute: 97,9% der Theorie — Brechungsindex $n_D^{25}$: 1,56oo

Beispiel 4 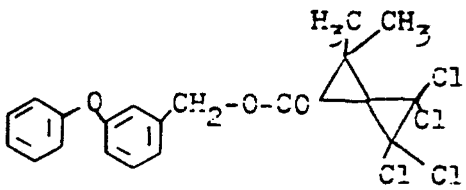 Ausbeute: 83,1% der Theorie — Brechungsindex $n_D^{25}$: 1,568o

Ausgangsverbindungen:

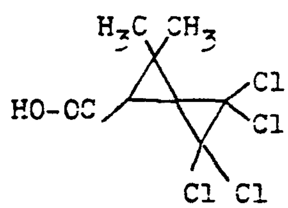

22,95 g (o,o75 Mol) 4,4,5,5-Tetrachloro-2,2-dimethyl-spiropentan-1-carbonsäure-methylester werden in 1oo ml Äthanol gelöst und nach Zugabe von 6 g Natriumhydroxid, gelöst in 1oo ml Wasser, 4 Stunden zum Rückfluß erhitzt. Anschließend wird das Äthanol im Vakuum abgezogen, der Rückstand mit 1oo ml Wasser versetzt und mit 2oo ml Methylenchlorid extrahiert. Die wässrige Phase wird mit konzentrierter Salzsäure angesäuert, der ausfallende Feststoff abgesaugt, mit Wasser gewaschen und getrocknet. Man erhält 13 g (62,4 % der Theorie) 4,4,5,5-Tetrachloro-2,2-dimethylspiropentan-1-carbonsäure als farb-

losen Feststoff mit dem Schmelzpunkt 198-199°C.

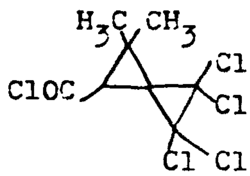

13 g (o,o467 Mol) 4,4,5,5-Tetrachloro-2,2-dimethylspiropentan-1-carbonsäure werden in 2oo ml Tetrachlorkohlenstoff gelöst und zusammen mit 5o ml Thionylchlorid 3 Stunden am Rückfluß erhitzt. Anschließend werden überschüssiges Thionylchlorid und Tetrachlorkohlenstoff im Vakuum abgezogen und der Rückstand im Vakuum destilliert. Man erhält 8,1 g (58,5 % der Theorie) 4,4,5,5-Tetrachloro-2,2-dimethylspiropentan-1-carbonsäurechlorid als farblose Flüssigkeit mit dem Siedepunkt 12o°C/1o Torr.

Patentansprüche

1. Spiropentancarbonsäureester der Formel (I)

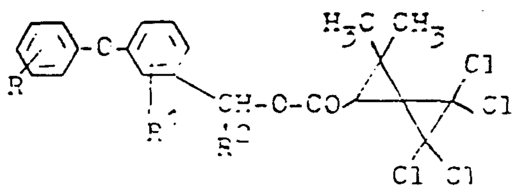

(I)

in welcher

R und $R^1$ gleich oder verschieden sind und für Wasserstoff oder Halogen stehen und

$R^2$ für Wasserstoff, Cyano oder Äthinyl steht.

2. Verfahren zur Herstellung der Spiropentancarbonsäureester der Formel (I), dadurch gekennzeichnet, daß man 4,4,5,5-Tetrachloro-2,2-dimethyl-spiropentancarbonsäurechlorid der Formel (II)

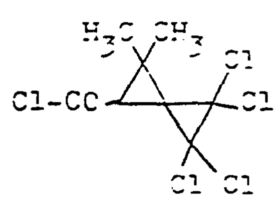

(II)

mit substituierten Phenoxybenzylalkoholen der Formel (III)

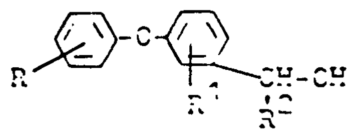

(III)

in welcher

R,$R^1$ und $R^2$ die oben angegebene Bedeutung haben,

gegebenenfalls in Gegenwart eines Säureakzeptors und gegebenenfalls in Gegenwart eines Verdünnungsmittels umsetzt.

3. Insektizide und akarizide Mittel, gekennzeichnet durch einen Gehalt an mindestens einem Spiropentancarbonsäureester der Formel (I) gemäß Anspruch 1.

4. Verwendung von Spiropentancarbonsäureester der Formel (I) gemäß Anspruch 1 zur Bekämpfung von Insekten oder Spinnentieren.

5. Verfahren zur Bekämpfung von Insekten oder Spinnentieren, dadurch gekennzeichnet, daß man Spiropentancarbonsäureester der Formel (I) gemäß Anspruch 1 auf Insekten oder Spinnentiere und/oder ihren Lebensraum einwirken läßt.

6. Verfahren zur Herstellung insektizider oder akarizider Mittel, dadurch gekennzeichnet, daß man Spiropentancarbonsäureester der Formel (I) gemäß Anspruch 1 mit Streckmitteln und/oder oberflächenaktiven Mitteln vermischt.

Europäisches Patentamt

# EUROPÄISCHER RECHERCHENBERICHT

0006456

Nummer der Anmeldung
EP 79 10 1626

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| A | DE - A - 2 713 651 (CIBA-GEIGY)<br>* Patentansprüche 1-7,8,9,10 und 11 * | 1,2,3, 4-6 |
| A | DE - A - 2 712 333 (SHELL)<br>* Patentansprüche 1 und 2,3,4 * | 1,2, 3-6 |
| D,A | DE - A - 2 621 433 (BAYER)<br>* Patentansprüche 1,2,3,4,5,6 * | 1,2,3, 4,5,6 |
| D,A | DE - A - 2 605 828 (AMERICAN CYANAMID)<br>* Patentansprüche 1-15, 16-26, 27 * | 1,2,3 |
| A | DE - A - 2 553 991 (SHELL)<br>* Patentansprüche 1-3,4 und 5,6 * | 1,2, 3-6 |
| | DE - A - 2 447 735 (SHELL)<br>* Beispiele 4 und 5, Patentansprüche 1-3, 10 und 11, 12 und 13 * | 1,2, 3-6 |

KLASSIFIKATION DER ANMELDUNG (Int.Cl.³)

C 07 C 69/74
A 01 N 9/24
C 07 C 121/68

RECHERCHIERTE SACHGEBIETE (Int. Cl.³)

C 07 C 69/74
C 07 C 121/68
A 01 N 9/24

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| WIEN | 9-08-1979 | REIF |